Europäisches Patentamt

European Patent Office

Office européen des brevets

(11) Publication number: **0 035 311**
**B1**

(12) **EUROPEAN PATENT SPECIFICATION**

(45) Date of publication of patent specification: **18.09.85**

(51) Int. Cl.⁴: **G 01 N 31/22**

(21) Application number: **81200223.6**

(22) Date of filing: **24.02.81**

(54) **A detector for detecting air components.**

(30) Priority: **25.02.80 NL 8001133**

(43) Date of publication of application:
**09.09.81 Bulletin 81/36**

(45) Publication of the grant of the patent:
**18.09.85 Bulletin 85/38**

(84) Designated Contracting States:
**AT BE CH DE FR GB IT LI LU NL SE**

(56) References cited:
**DE-A-2 062 710**
**DE-A-2 647 258**
**US-A-3 482 944**
**US-A-3 809 617**

(73) Proprietor: **DUPHAR INTERNATIONAL
RESEARCH B.V**
**C.J. van Houtenlaan 36**
**NL-1381 CP Weesp (NL)**

(72) Inventor: **Heijenga, Berend**
**Schaapstreek 42**
**NL-7841 BS Sleen (NL)**
Inventor: **Hilbrink, Hubertus Eduard**
**Tingietersdonk 225**
**NL-7326 NK Apeldoorn (NL)**
Inventor: **Klaij, Henri Frits**
**Malmbrink 79**
**NL-7544 LG Enschede (NL)**

(74) Representative: **Mommaerts, Johan Hendrik,
Dipl.-Phys.**
**Octrooibureau Lux Willem Witsenplein 4**
**NL-2596 BK Den Haag (NL)**     -

Courier Press, Leamington Spa, England.

## Description

The invention relates to a detector for detecting the presence of poisonous or noxious components in the air, comprising a housing, provided with air passages, in which an air pervious carrier containing a first reagent is arranged, through which carrier the air to be tested is to be sucked in order to expose this reagent to the air, which reagent is influenced by the components to be detected, which carrier is to be brought into contact with a second reagent which, together with the first one, can bring about a colour reaction depending on whether or not the first reagent has been influenced, said housing further being an integral unit whilst in an operative condition during which said air is being sucked through said carrier and comprising an air- and liquid-tight container, having an easily rupturable wall arranged within said housing and near the carrier for the first reagent, and filled with uncontaminated reagent or dissolving liquid. Such a detector is known from DE—A 2 647 258 of the same applicant, in which the housing is a glass tube comprising a glass ampulla containing said liquid, and after breaking the tube ends air can be led through the reagent carriers provided in said tube. The reaction can be brought about by breaking the ampulla so that the reagent carriers are moistened by the liquid.

This known detector has a number of disadvantages. Because of the small cross-section of the tube and the relatively high flow resistance of the relatively thick reagent carriers and intermediate porous spacers, the air volume led through per unit of time will be small. Moreover, such tubes are not suitable for being mounted on the suction opening of a gas mask, which would substantially simplify its use as a gas detector for determining whether the outside conditions still require the use of a gas mask. Furthermore it is very difficult to handle such detectors when wearing thick and heavy gloves which is generally the case when working in poisoned or contaminated areas.

The invention provides an improved gas detector not having these disadvantages, which gas detector is, in particular, suitable for being placed on a gas mask so as to simplify the step of leading through the air to be tested, and which can be easily manipulated with gloved hands.

The detector according to the invention is characterised in that the housing is a flat box-shaped closed housing with a substantially circular periphery, in that the first reagent carrier is a sheet of filter paper, in that the liquid container is a disc-shaped container made of foil material, in that an inwardly yieldable flat wall portion of the housing is situated near said container, and is adapted to be brought into contact with said container, and in that a plurality of piercing pins is provided in such a manner that, when said wall portion is pushed inwardly of said housing, the container wall is ruptured so as to moisten the reagent carrier with the liquid from said container.

Such a detector can be held between the thumb and index finger of one hand, and the wall portion can be pushed inwards by said fingers, even when wearing heavy or thick gloves. Moreover the flow cross-section for the air led through is large, and the reagent carrier has a large surface area and a small thickness, so that the air can be sucked through quickly, and the reagent is contacted therewith over the full extent of the reagent carrier.

From a still older proposal of the same application as disclosed in DE—A 2 062 710 a gas detector is known, comprising a relatively flat box-like housing made of two interfitting halves each provided with a reagent carrier in the form of sheets of filter paper. After sucking air through one carrier, it is to be wetted, and then both carriers are to be contacted by closing the box. The disadvantage is that a liquid from an external source, which can be contaminated, is to be used, and that handling such a two part housing with gloved hands is very difficult, and both hands are needed for using such a detector.

In particular the second reagent, which will produce with the first one a colour reaction, will be provided on or in a second plane carrier, which is normally kept separated from the first carrier, and is, in particular, made of filter paper, which carriers are to be contacted with one another and are to be moistened with a suitable liquid, and according to the invention, the second carrier is supported a small distance from and parallel to the first one inside said housing, this in such a manner that, when pressing said yieldable wall portion inwards, both carriers are contacted with one another, the container being filled with a suitable moistening agent, and in particular with pure water.

Such a detector takes very little space, since the housing, in the operative condition, consists of one integral piece, which moreover, facilitates handling, as the only intervention to be made is pressing inwards the yieldable wall portion, which can be done in a simple manner also with the gloved hand. The whole assembly is enclosed in an envelope protecting against environmental influences, which can be easily ruptured also with the gloved hand, so that such detectors can be stored for a long time without losing their activity. A special security against influences disturbing the dependability is obtained because the moistening liquid, which may contain, if desired, the second reagent, is comprised in a fully shielded manner in a separate container. Packing liquids in a sterile manner is known from the pharmaceutical industry, and does not provide special difficulties.

In particular the housing is provided with at least one collar or seat fitting in the air suction opening of a gas mask or gas mask filter, so that sucking air through the first reagent carrier will be brought about in a very simple manner. This seat can, for instance, be shaped as a conical surface fitting in openings of different diameter so as to provide an adaptation to different gas mask types, and it is also possible to provide a special seat for non-regular gas mask types, for instance at the other side of the housing. It is also possible to fix such a detector to a stick or the like, and to swing it through the air so as to obtain the required amount of air flowing through. This will, in particular, be done if, for instance, the contents of a storage vessel or space are to be investigated from the outside.

In an embodiment which can easily be manufactured the first reagent carrier is clamped between a bottom rim of the housing and an insert, the latter being provided with support surfaces for supporting the liquid container and, as the case may be, a second reagent carrier, said insert being adapted to be fixed in the housing by means of a clamping ring, and, in particular, the yieldable wall part is connected to said clamping ring by means of yielding and, if required, rupturable lips. Such parts can be manufactured in a simple manner from plastics, and can be assembled very quickly.

The piercing pin or pins are, in particular, provided at the bottom of the housing, which bottom is provided with air passages, which pins are, in particular, provided on the spoke-like wall portions between said passages.

In order to prevent the unintentional rupturing of the liquid container, a removable lid is preferably provided on the housing which, in particular, tightly fits on the rim of the housing.

In order to protect the reagents provided on a carrier against regression, it is advisable to provide a drying means which, in particular, is shaped as a flat ring or disc consisting of or comprising an absorbing material, which, in particular, can be situated within the protecting lid, and, for instance, can annularly surround the yieldable wall portion. Such a ring will, moreover, provide an additional protection of the yieldable wall portion since the forces exerted on the protective lid which, itself, generally consists of a thin plastic plate, can be diverted thereby towards the housing.

Such a detector can be used, besides for the purposes mentioned in the above-mentioned prior patent application, also for detecting any component in air for which a suitable reagent exists.

The invention will be elucidated below in more detail by reference to a drawing, showing in:

Figs. 1 and 2 a cross section and an exploded view respectively of a first embodiment of the detector of the invention; and

Fig. 3 a cross section of a second embodiment thereof.

The gas detector shown in the drawing comprises a box-shaped housing 1 with a bottom 2 with a circular periphery joining a conical lateral wall 3. The bottom 2 comprises uniformly distributed air passages 4, and the intermediate spokes are provided with piercing pins 5.

Inside the housing 1 an insert 6 is placed, having oblique lips 7 bearing against the lateral wall 3 of the housing 1, and being mutually separated by gaps 8, which lips ensure a good fitting against the lateral wall 3. The lower edges 9 of the lips 7 fit in a circumferential groove 10 of the bottom 2. The insert 6 is, furthermore, provided with a plurality of inwardly directed lateral lips 11 with a plane upper surface.

On the insert 6 bears a clamping ring 13 which is fixed on the housing 1 behind an incision 14. A pressure plate 15 is connected to the ring 13 by means of a plurality of yieldable and, as the case may be, rupturable lips 16, between which lips air openings are present.

On the rim of the bottom 2 lies a piece of reagent paper 18 which, furthermore, is pressed by the lip 7 of the insert 6 into the groove 10. On the lateral lips 11 of the insert 6 lies a second piece of reagent paper 19 which is maintained by the insert 6 at some distance from the reagent paper 18.

The reagent paper 18 consists, for example, of filter paper or similar absorbent carrier material with a low air flow resistance, containing the enzyme butyryl cholesterinase, the reagent paper 19 containing, for instance, 2.6-dichloro-indophenyl acetate. This enzyme is sensitive for certain poisonous substances, such as, for instance, substances used as insecticides, nerve gases and the like, which substances inactivate the enzyme. The second reagent will be discoloured under the influence of the active enzyme, so that, from the colour the presence of the enzyme attacking substances can be derived. As a colour reagent also a mixture of, for instance, α-naphtyl acetate and diazonium blue can be used. All this has already been described in DE—A—2062710. Of course similar colour reactions can also be obtained by means of other reagents which are sensitive for similar or other components present in the air.

On the surface 12 of the lateral lips 11 rests a gas- and liquid-tight liquid container 20 consisting of a gas- and liquid-tight foil material, e.g. metallic foil coated with plastics or the like. This container is filled with a liquid required for or enhancing the reaction, and can in the considered case consist of, for instance, pure water which is then to be used for moistening both reagent carriers 18 and 19 so as to bring about hydrolysis, or to mix the reagents with one another.

Around the pressure plate 15 and on a plane collar portion inside an upright flange 22 of the housing rests a disc of moisture absorbing material, e.g. paper or textile, impregnated with silica gel. This assembly is covered by means of a protective lid 24 having a lateral rim 25 which tightly fits on the flange 22 of the housing 1. This lid prevents that the pressure plate 15 will be untimely pressed inwards. Moreover, the discs 23 can divert the forces exerted thereon towards the housing 1 when the lid 24 is pressed inwards.

The housing 1 filled and closed in this manner is enclosed in a gas- and liquid-tight envelope 26 made of metallic foil and/or plastics which is closed by means of a cover layer 27, which cover layer can be pulled away by means of a pulling tab 28. In this manner the gas detector is kept separated from the surroundings in a gas- and liquid-tight manner, and the drying means 23 keeps the interior of the envelope dry. The lid 24 protects the liquid container 20 against damage for forces exerted on the whole assembly.

In order to use the gas detector, at first the envelope 26 is pulled open, after which the lid 24 and the drying means 23 are removed. This can be done with gloved hands, since the pulling tab 28 and the lateral flange 25 of the lid 24 provide sufficient hold.

The housing 1 is, then, arranged in the suction opening of a gas mask filter box, either with the conical wall 3 or with the flange 22. The conical wall 3 is, for instance, suitable for the current European gas masks, and the flange 22 is adapted to American gas masks. In this manner one single detector is made suitable for all the gas mask types now currently in use.

When breathing, the air is sucked inwards through the reagent paper 18 in which the components to be detected are absorbed. After a certain suction time the detector is removed again from the gas mask, and then the pressure plate 15 is pressed inwards. The liquid container is, then, pierced by the pins 5 so that the reagent paper 18 and 19 are moistened. At the same time these papers are pressed against one another. After a given reaction time it can be established on the basis of the colour change whether the threshold concentration of the components in question has been exceeded.

Such detectors are not very voluminous and are very light, so that the user of a gas mask can take with him a sufficient number thereof. Since the detectors contain all the components required for the reaction, including the moistening liquid, no other interventions are required than opening the envelope and rupturing the liquid container, and finally sucking through a given amount of air with the aid of a gas mask.

The detector according to the invention comprises a small number of parts which can be manufactured and assembled in a simple manner, so that it becomes possible to manufacture large numbers thereof at a relatively low price. Moreover, such detectors are enclosed in such a manner that they are tenable very long.

It is also possible to mount such a detector, e.g. in a suitable seat, on a rod, and to swing it through the air to be tested. This can, for instance, be done for testing the contents of tanks or storage spaces in which harmful gases or vapours can be present, and the rod can be inserted inwardly through a manhole or door opening.

Such a detector can also be used with other reagents than those mentioned above, for instance for being used to detect other air components. It is, for instance, also possible to use only one reagent paper 18, and to dissolve the other reagent bringing about a colour reaction in the liquid in the liquid container 20. Furthermore it is possible to provide both reagents in one piece of filter paper if they do not react in the dry condition, which is made possible, in particular, since the contents of the envelope 26 are kept dry. Such a detector can also be used, for instance, for detecting alcohol in exhaled air and for similar purposes.

It is, for the rest, not always necessary to enclose such detectors in an envelope 26, and it is often also possible to enclose a plurality of detectors together in a moisture- and gas-tight manner.

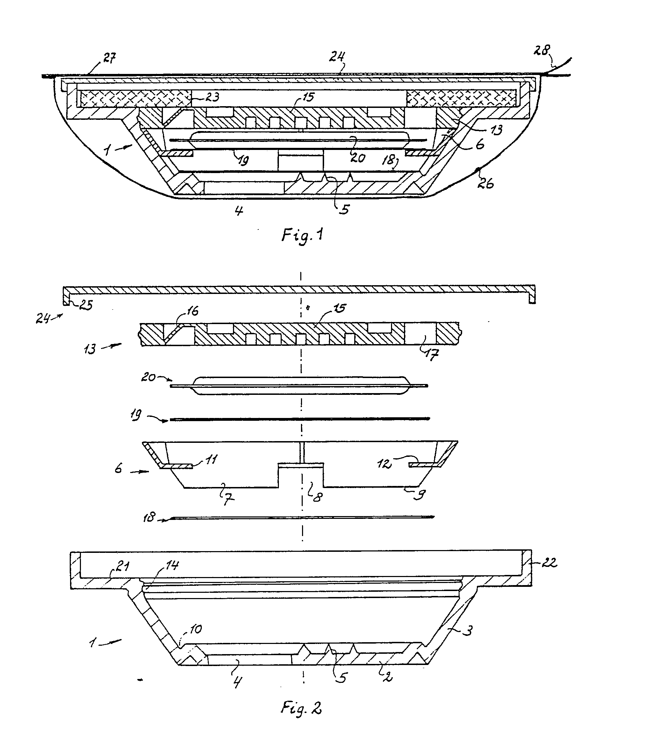

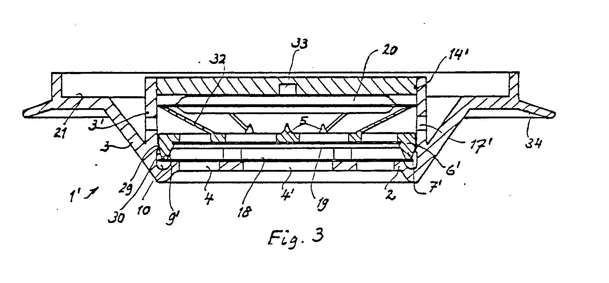

The embodiment shown in Fig. 3 mainly corresponds to that of Figs. 1 and 2. Similar parts have been indicated by the same reference numerals, and modified parts by primed ones.

The housing 1' comprises an internal cylindrical sleeve or barrel 3' which is integrally connected to the conical outer wall 3, in which sleeve the essential elements are arranged. The bottom of the housing 1' is provided with an additional central opening 4' for improving the visibility of the reagent paper 18. The pins 5 are no longer connected to the bottom 2, as will be described below.

The insert 6' of this embodiment is provided with a plurality of teeth 7' which are narrower than the lips 7 of Figs. 1 and 2. Moreover, in the normal position shown, the extremities 9' of the teeth 7' do not extend into the circumferential groove 10, since the insert 6' is maintained in the position shown by means of a detent ridge 29 bearing on chamfered surface parts 30 of the teeth 7'. The lower faces 9' of the teeth 7' keep the reagent paper 18 slightly pressed against the upper surface of the bottom 2.

The second reagent paper 19 is supported by the insert 6' which is, for instance, provided with a circumferential groove 31 for fixing the sheet 19. Alternatively, the insert may comprise two parts between which a sheet of reagent paper can be clamped.

The piercing pins 5 are, now, provided on the insert 6' at the upper side thereof, so that it is no longer necessary, as in the first embodiment, to pierce also two layers of reagent paper before piercing the container 20. In order to prevent that the latter will touch the pins 5, the insert 6' is provided with a plurality of resilient and/or rupturable fingers 32, and the rim part of the container 20 bears on the extremities of these fingers, the container thus being kept at a suitable distance from the sharp points of the pins 5.

The pressure plate 15 is, now, directly connected to the sleeve 3' by means of a snap lock 14'. The fingers 32 press the container 20 against the lower surface of this plate 15.

When pressing the plate 15 inwards, the snap lock 14' is released, and the container is pressed against the fingers 32. The latter will yield or break, and then the container 20 is pressed against the pins 5 which will pierce the container wall, and will, eventually, enter recesses 33 in the plate 15. Subsequently the insert 6' is pressed downwards, and then the teeth 7' will slightly be pressed inwards so as to slide past the ridge 29.

The sheet 18 is tightened by the teeth 7' entering the groove 10, and both sheets 18 and 19 will then be contacted with one another.

The pressure plate 15 shown has no air passages 18 as in the case of Figs. 1 and 2, but air passages 17' are provided in the sleeve 3'. However, it is also possible to provide passages 17 in the plate 15.

The housing 1' is provided with an extending circumferential rim 34, which serves as a handle when placing the detector on a gas mask, and instead thereof one or more discrete handle parts can be provided. It will be clear that such parts can also be provided in the case of Figs. 1 and 2.

It will be clear that the pins 5 can also be provided on the plate 15, which should, then, also be provided with the fingers 32, and then a simpler insert can be used.

Many other modifications are possible, and in particular features of the embodiment of Fig. 3 may be used in the embodiment of Figs. 1 and 2 and vice versa.

## Claims

1. A detector for detecting the presence of poisonous or noxious components in the air, comprising a housing (1), provided with air passages (4, 4', 17, 17'), in which an air pervious carrier (18) containing a first reagent is arranged, through which carrier (18) the air to be tested is to be sucked in order to expose this reagent to the air, which reagent is influenced by the components to be detected, which carrier (18) is to be brought into contact with a second reagent which, together with the first one, can bring about a colour reaction depending on whether or not the first reagent has been influenced, said housing (1) being an integral unit whilst in an operative condition during which said air is being sucked through said carrier and further comprising an air- and liquid-tight container (20), having an easily rupturable wall arranged within said housing and near the carrier (18) for the first reagent, and filled with uncontaminated reagent or dissolving liquid, characterised in that the housing (1) is a flat box-shaped closed housing with a substantially circular periphery, in that the first reagent carrier (18) is a sheet of filter paper, in that the liquid container (20) is a disc-shaped container made of foil material, in that an inwardly yieldable flat wall portion (15) of the housing (1) is situated near said container (20), and is adapted to be brought into contact with said container (20), and in that a plurality of piercing pins (5) is provided in such a manner that, when said wall portion (15) is pushed inwardly of said housing, the container wall is ruptured so as to moisten the reagent carrier (18) with the liquid from said container (20).

2. The detector of claim 1, characterised in that the housing (1) is enclosed by a gas- and liquid-tight and rupturable envelope (26) of foil material.

3. The detector of claim 1 or 2, in which the second reagent is applied on a second carrier (19) separated from the first one (18) which carriers (18, 19) are adapted to be brought into contact with one another, and are to be moistened by the liquid from the container (20) for bringing out the reaction, characterised in that the second carrier in the form of a second sheet of filter paper (19) is supported within the housing at a small distance of the first carrier (18), and this in such a manner that, when pressing inwards the yieldable wall portion (15), said carriers will be brought into contact with one another.

4. The detector of claim 1 or 2, characterised in that the second reagent is comprised in the liquid in the container (20).

5. The detector of any one of claims 1 to 4, characterised in that the housing (1) is provided with at least one collar or seat fitting in the air suction opening of a gas mask or gas mask filter.

6. The detector of claim 5, characterised in that the seat is in the shape of a conical surface (3) fitting in openings of different diameters.

7. The detector of any one of claims 1 to 6, characterised by means for mounting it on a rod or the like to be inserted into a space to be tested.

8. The detector of any one of claims 1 to 7, characterised by an insert (6, 6') adapted to clamp the first reagent carrier (18) against a bottom rim (10) of the housing (1), which insert (6, 6') is provided with supporting surfaces (11, 32) for supporting the liquid container (20), and as the case may be, the second reagent carrier (19), and by clamping means (13) for fixing the insert (6, 6') in the housing (1).

9. The detector of claim 8, characterised in that the yieldable wall portion (15) is part of the clamping means (13).

10. The detector of claim 9, characterised in that the yieldable wall portion (15) is connected to the clamping means (13) by means of yieldable and, as the case may be, rupturable lips (16).

11. The detector of any one of claims 1 to 10, characterised in that the piercing pins (5) are arranged on the housing bottom (2) provided with air passages (4).

12. The detector of any one of claims 1 to 10, characterised in that the piercing pins (5) are arranged on the yieldable wall portion (15).

13. The detector of any one of claims 8 to 10, characterised in that the piercing pins (5) are provided on the insert (6').

14. The detector of any one of claims 1 to 13, characterised by resilient and/or rupturable support means (32) for the container (20).

15. The detector of any one of claims 8 to 14, characterised in that the clamping means (29) for the insert (6') allow it to be displaced towards the first reagent carrier (18) by the yieldable wall portion (15).

16. The detector of any one of claims 1 to 15, characterised by a safety cover (24) which is removably arranged on the housing (1), and is adapted for shielding the yieldable wall portion (15).

17. The detector of claim 16, characterised in that the cover (24) is provided with a clamping rim (25) tightly fitting on the rim (22) of the housing (1).

18. The detector of any one of claims 2 to 17, characterised by a drying agent provided within the housing (1).

19. The detector of claim 18, characterised in that the drying agent is provided in a flat ring or disc (23) made of an absorbing material.

20. The detector of claim 19, characterised in that the ring (23) containing the drying agent is situated within the protecting lid (24), and surrounds the yieldable wall portion (15).

21. The detector of any one of claims 1 to 20, characterised in that the housing (1) is provided with outwardly extending handle means.


**Patentansprüche**

1. Detektor zum Feststellen der Anwesendheit eines giftigen oder schädlichen Bestandteiles in der Luft, mit einem Gehäuse (1), das im Gebrauchszustand eine zusammenhängende Einheit bildet und mit Luftdurchlässen (4, 4', 17, 17') versehen ist, im welchem ein luftdurchlässiger Träger (18), der ein erstes Reagens enthält, vorgesehen ist, durch welchen Träger (18) hindurch die zu untersuchende Luft hindurchgesaugt wird, um dadurch dieses Reagens der Luft auszusetzen, welches Reagens von den festzustellenden Bestandteilen beeinflusst wird, welcher Träger (18) mit einem zweiten Reagens in Berührung zu bringen ist, das zusammen mit ersterem, abhängig von der Tatsache, ob das erste Reagens wohl oder nicht beeinflusst worden ist, eine Farbreaktion herbeiführen kann, welches Gehäuse (1) weiterhin einen luft- und flüssigkeitsdichten Behälter (20) mit einer leicht zerreissbaren Wand enthält, der innerhalb dieses Gehäuses und in der Nähe des Trägers (18) für das erste Reagens angeordnet ist, und der mit einem nicht verunreinigtem Reagens oder Lösungsflüssigkeit gefüllt ist, dadurch gekennzeichnet, dass das Gehäuse (1) eine flaches geschlossenes Gehäuse mit in der Hauptsache kreisförmigen Umriss ist, dass der erste Reagensträger (18) eine Blatt Filterpapier ist, dass der Flüssigkeitsbehälter (20) eine scheibenförmiger Behälter aus Foliematerial ist, dass ein nach innen nachgiebiger flacher Wandteil (15) des Gehäuses (1) in der Nähe dieses Behälters (20) vorgesehen ist und mit diesem Behälter (20) in Berührung gebracht werden kann, und dass eine Anzahl von Durchstechzapfen (5) in solcher Weise angeordnet ist, dass, wenn dieser Wandteil (15) nach innen gedruckt wird, die Behälterwand zerrissen wird, um dadurch den Reagensträger (18) mit der Flüssigkeit aus diesem Behälter (20) zu benetzen.

2. Detektor nach Anspruch 1, dadurch gekennzeichnet, dass das Gehäuse (1) in einer gas- und flüssigkeitsdichten Hülle (26) aus Foliematerial aufgenommen ist.

3. Detektor nach Anspruch 1 oder 2, in welchem das zweite Reagens auf einem zweiten, vom ersteren Träger (18) getrennten Träger (19) aufgebracht ist, welche Träger (18, 19) miteinander in Berührung gebracht werden können, und mit der Flüssigkeit aus dem Behälter (20) benetzt werden müssen um die Reaktion herbeizuführen, dadurch gekennzeichnet, dass der zweite Träger in der Form eines zweiten Filterpapierblattes (19) innerhalb des Gehäuses in geringerer Entfernung vom ersteren Träger (18) unterstützt ist, dies in solcher Weise, dass, wenn der nachgiebige Wandteil (15) nach innen gedruckt wird, die beiden Träger (18, 19) miteinander in Berührung gebracht werden.

4. Detektor nach Anspruch 1 oder 2, dadurch gekennzeichnet, dass das zweite Reagens in der Flüssigkeit im Behälter (20) aufgenommen ist.

5. Detektor nach einem der Ansprüche 1—4, dadurch gekennzeichnet, dass das Gehäuse (1) mit wenigstens eine, Kragen oder Sitz versehen ist, der in der Luftansaugöffnung einer Gasmaske oder eines Gasmaskenfilters passt.

6. Detektor nach Anspruch 5, dadurch gekennzeichnet, dass der Sitz die Gestalt einer Kegelfläche (3) hat, die in Öffnungen vershiedenen Durchmessers passt.

7. Detektor nach einem der Ansprüche 1—6, gekenzeichnet durch Mittel zur Befestigung auf einem Stab od.dgl., welcher in einen zu untersuchenden Raum hineingestreckt werden kann.

8. Detektor nach einem der Ansprüche 1—7, gekennzeichnet durch eine Einsatzteil (6, 6'), das den ersten Reagensträger (18) an einem Bodenrand (10) des Gehäuses (1) klemmen kann, welches Einsatzteil (6, 6') mit Stützflächen (11, 32) zum Unterstützen des Flüssigkeitsbehälters (20) und ggf. des zweiten Reagensträgers (19) versehen ist, und durch Klemmenmittel (13) zum Festhalten des Einsatzteiles (6, 6') im Gehäuse (1).

9. Detektor nach Anspruch 8, dadurch gekennzeichnet, dass der nachgiebige Wandteil (15) eine Teil des Klemmittels (13) ist.

10. Detektor nach Anspruch 9, dadurch gekennzeichnet, dass der nachgiebige Wandteil (15) mittels nachgiebigen und ggf. zerreissbaren Zungen (16) mit dem Klemmittel (13) verbunden ist.

11. Detektor nach einem der Ansprüche 1—10, dadurch gekennzeichnet, dass die Durchstechzapfen (5) am mit Luftdurchlässen (4) versehenen Gehäuseboden angeordnet sind.

12. Detektor nach einem der Ansprüche 1—10, dadurch gekennzeichnet, dass die Durchstechzapfen (5) am nachgiebigen Wandteil (15) angeordnet sind.

13. Detektor nach einem der Ansprüche 8—10, dadurch gekennzeichnet, dass die Durchstechzapfen (5) am Einsatzteil (6') angeordnet sind.

14. Detektor nach einem der Ansprüche 1—13, gekennzeichnet durch nachgiebige und/oder zerreissbare Stützmittel (32) für den Behälter (20).

15. Detektor nach einem der Ansprüche 8—14, dadurch gekennzeichnet, dass die Klemmittel (29) für das Einsatzteil (6°) mittels des nachgiebigen Wandteiles (15) auf den ersten Reagensträger (18) hin bewegbar sind.

16. Detektor nach einem der Ansprüche 8—14, gekennzeichnet durch einen Sicherheitsdeckel (24), der lösbar am Gehäuse (1) angeordnet ist, und zum Schützen des nachgiebigen Wandteiles (15) geeignet ist.

17. Detektor nach Anspruch 16, dadurch gekennzeichnet, dass der Deckel (24) mit einem fest auf den Rand (22) des Gehäuses (1) passenden Klemmring (25) versehen ist.

18. Detektor nach einem der Ansprüche 2—17, gekennzeichnet durch ein innerhalb des Gehäuses (1) vorgesehenes Trockenmittel.

19. Detektor nach Anspruch 18, dadurch gekennzeichnet, dass das Trockenmittel in einem flachen Ring oder Scheibe (23) aus absorbierendem Material aufgenommen ist.

20. Detektor nach Anspruch 19, dadurch gekennzeichnet, dass der das Trockenmittel enthaltende Ring (23) innerhalb des Schutzdeckels (24) angeordnet ist, und den nachgiebigen Wandteil (15) umgibt.

21. Detektor nach einem der Ansprüche 1—20, dadurch gekennzeichnet, dass das Gehäuse (1) mit nach aussen gerichteten Handhabemitteln versehen ist.

## Revendications

1. Détecter pour détecter la présence de composants toxiques ou nocifs dans l'air, comprenant un boîtier (1), prévu avec des passages d'air (4, 4', 17, 17'), dans lequel est disposé un élément porteur (18), perméable à l'air, contenant un premier réactif et à travers lequel l'air à contrôler doit être aspiré afin d'exposer ce réactif à l'air, ledit réactif étant sensible aux composants à détecter, l'élément porteur (18) devant être mis en contact avec un deuxième réactif qui, combiné au premier réactif, peut engendrer une réaction colorée selon que le premier réactif a été influencé ou non par les dits composants, le boîtier (1) étant une unité monobloc en situation de fonctionnement, pendant laquelle l'air est aspiré à travers l'élément porteur, et comprenant en outre un récipient (20), étanche à l'air et au liquide, qui comporte un paroi facile à briser située à l'intérieur du boîtier et près de l'élément porteur (18) pour le premier réactif et qui est rempli d'un réactif non contaminé ou d'un liquide de dissolution, caractérisé en ce que le boîtier (1) est un boîter fermé en forme de boîte plate à périphérie sensiblement circulaire, en ce que l'élément porteur (18) du premier réactif est une feuille de papier filtrant, en ce que le récipient (20) de liquide est un récipient à paroi mince en forme de disque, en ce qu'une partie de paroi plane (15), déformable vers l'intérieur, du boîtier (1) est située près du récipient (20) et est prévue pour être mise en contact avec le récipient (20), et en ce qu'une pluralité de saillies de perçage (5) sont prévues de sorte que, lorsque la partie de paroi (15) est poussée vers l'intérieur du boîtier, la paroi du récipient est brisée de façon à humidifier l'élément (18) porteur de réactif avec le liquide du récipient (20).

2. Détecteur suivant la revendication 1, caractérisé en ce que le boîtier (1) est enfermé dans une enveloppe déchirable (26) en matière mince, étanche aux gaz et aux liquides.

3. Détecteur suivant la revendication 1 ou 2, dans lequel le deuxième réactif est appliqué sur un deuxième élément porteur (19) séparé du premier (18), ces éléments porteurs (18, 19) étant prévus pour être mis en contact l'une avec l'autre et devant être humidifiés par le liquide du récipient (20) pour engendrer la réaction, caractérisé en ce que le deuxième élément porteur, sous la forme d'une deuxième feuille de papier filtrant (19), est supporté dans le boîtier à une petite distance du premier élément porteur (18), et cela d'une manière telle que, lorsqu'on presse vers l'intérieur la partie de paroi déformable (15), les éléments porteurs sont mis en contact l'un avec l'autre.

4. Détecteur suivant la revendication 1 ou 2, caractérisé en ce que le deuxième réactif est inclus dans le liquide du récipient (20).

5. Détecteur suivant l'une quelconque des revendications 1 à 4, caractérisé en ce que le boîtier (1) comporte au moins une collerette ou un siège s'ajustant dans l'orifice d'aspiration d'air d'un masque à gaz ou d'un filtre de masque à gaz.

6. Détecteur suivant la revendication 5, caractérisé en ce que le siège a la forme d'une surface conique (3) se logeant dans les orifices de diamètre différent.

7. Détecteur suivant l'une quelconque des revendications 1 à 6, caractérisé par des moyens permettant son montage sur une tige ou dispositif analogue à insérer dans un espace à contrôler.

8. Détecteur suivant l'une quelconque des revendications 1 à 7, caractérisé un insert (6, 6') prévu pour bloquer l'élément porteur (18) du premier réactif contre un siège inférieur (10) du boîtier (1), cet insert (6, 6') comportant des surfaces d'appui (11, 32) pour supporter le récipient de liquide (20) et, le cas échéant, l'élément porteur (19) du deuxième réactif, et par des moyens de serrage (13) pour fixer l'insert (6, 6') dans le boîtier (1).

9. Détecteur suivant la revendication 8, caractérisé en ce que la partie de paroi déformable (15) est une partie des moyens de serrage (13).

10. Détecteur suivant la revendication 9, caractérisé en ce que la partie de paroi déformable (15) est reliée aus moyens de serrage (13) par des lèvres (16) déformables et, le cas échéant, cassables.

11. Détecteur suivant l'une quelconque des revendications 1 à 10, caractérisé en ce que les saillies de perçage (5) sont prévues sur le fond (2) du boîtier qui comporte des passages d'air (4).

12. Détecteur suivant l'une quelconque des revendications 1 à 10, caractérisé en ce que les saillies de perçage sont prévues sur la partie de paroi déformable (15).

13. Détecteur suivant l'une quelconque des revendications 8 à 10, caractérisé en ce que les saillies de perçage (5) sont prévues sur l'insert (6').

14. Détecteur suivant l'une quelconque des revendications 1 à 13, caractérisé par des supports (32) élastiques et/ou cassables pour le récipient (20).

15. Détecteur suivant l'une quelconque des revendications 8 à 14, caractérisé en ce que les moyens de serrage (29) pour l'insert (6') permettent son déplacement vers l'élément porteur (18) du premier réactif, par la partie de paroi déformable (15).

16. Détecteur suivant l'une quelconque des revendications 1 à 15, caractérisé par un couvercle de sécurité (24) qui est placé de façon amovible sur le boîtier (1), et qui est prévu pour protéger la partie de paroi déformable (15).

17. Détecteur suivant la revendication 16, caractérisé en ce que le couvercle (24) comporte un rebord de serrage (25) s'ajustant étroitement sur le bord (22) du boîtier (1).

18. Détecteur suivant l'une quelconque des revendications 2 à 17, caractérisé par un agent de dessication prévu dans le boîtier (1).

19. Détecteur suivant la revendication 18, caractérisé en ce que l'agent de dessication est prévu dans un anneau ou un disque plat (23) en matière absorbante.

20. Détecteur suivant la revendication 19, caractérisé en ce que l'anneau (23) contenant l'agent de dessication est situé à l'intérieur du couvercle de protection (24) et entoure la partie de paroi déformable (15).

21. Détecteur suivant l'une quelconque des revendications 1 à 20, caractérisé en ce que le boîtier (1) est muni d'une poignée s'étendant extérieurement.

Fig. 1

Fig. 2

1

Fig. 3